# EUROPEAN PATENT APPLICATION

(11) **EP 3 473 650 A1**
(43) Date of publication of application: **24.04.2019**
(21) Application number: 17197139.3
(22) Date of filing: 18.10.2017
(51) Int. Cl.: C07K 16/38

(54) **ANTIBODY FOR THE SPECIFIC DETECTION OF CAAP47/48 FRAGMENTS**

(71) Applicant: Universitätsklinikum Jena, 07747 Jena (DE); Leibniz-Institut für Naturstoff-Forschung und Infektionsbiologie - Hans-Knöll-Institut, 07745 Jena (DE)
(72) Inventor: Kiehntopf, Dr. Michael, 07743 Jena (DE); Schmerler, Dr. Diana, 07743 Jena (DE); Kaczmarek, Anika, 16356 Werneuchen (DE); Horn, Dr. Uwe, 99707 Kyffhäuserland (DE)
(74) Representative: CH Kilger Anwaltspartnerschaft mbB

(57) **Abstract**

The present invention relates to an antibody that binds specifically to CAAP47/48 fragments, allowing for the highly specific detection of CAAP47/48 for diagnostic purposes.

The invention further relates to a vector and kit comprising said antibody. The antibody can be used for determining and/or differentially diagnosing a CAAP47/48 correlated disease. For example, the antibody according to the invention can be used for determining whether an infection is of bacterial or non-bacterial origin.

The present invention also relates to a method for determining in a patient whether an infection is of bacterial or non-bacterial origin using the antibody according to the invention. Said method comprises determining the concentration of CAAP47/48 in a sample that has been taken from said patient, and determining whether said patient suffers from an infection of bacterial or non-bacterial origin based on the concentration of CAAP47/48 and designating the infection as of bacterial or non-bacterial origin.

Another aspect of the present invention is a method of monitoring the treatment of a patient with an antimicrobial drug, comprising determining the level of CAAP47/48 in a sample taking from said patient by using the antibody according to the invention and evaluating the effect of treatment with said antimicrobial drug based on the comparison of the levels of CAAP47/48 to reference values and the level of CAAP47/48 at different time-points in the same patient.

## Description

### Field of Invention

The present invention is in the field of medicine. It relates to an antibody that binds specifically to CAAP47/48 fragments, allowing for the highly specific detection of CAAP47/48 for diagnostic purposes. The invention further relates to a vector encoding said antibody and kit comprising said antibody as well as to a method for diagnosing in a patient a disease in which the levels of CAAP47/48 are altered.

### Background

A major factor underlying the abuse of antibiotics is the lack of rapid and accurate diagnostic tests for the early discrimination of bacterial versus non-bacterial infections. Proper diagnosis still remains a challenge in clinics which is mainly due to the fact that the clinical symptoms of infections from different causative agents can be very similar. For example, it may be difficult in certain instances to differentiate bacterial from viral or fungal infections.

Among others, procalcitonin (PCT) and C-reactive protein (CRP) markers are commonly used for diagnosing a bacterial infection and/or differentiating between different types of infection or systemic inflammatory reactions. However, although PCT and CRP-Tests are prevalent in clinical settings, their accuracy is heavily questioned and varies between different studies. Also, the clinical benefit for diagnostics and monitoring of therapy is debated as costs for tests are very high. Therefore, up to date there is still no reliable biomarker in clinical application which facilitates the differential diagnoses between different types of infection or systemic inflammatory reactions.

Previously, the inventors identified the proteolytic fragments (CAAP47/48) of alpha-1-antitrypsin (AAT) as such a potential reliable biomarker (Blaurock et al., Mediators of inflammation, 2016). The inventors showed that CAAP47/48 allows the discrimination of patients suffering from a bacterial infection, from patients with an infection of non-bacterial origin with very high sensitivity and specificity.

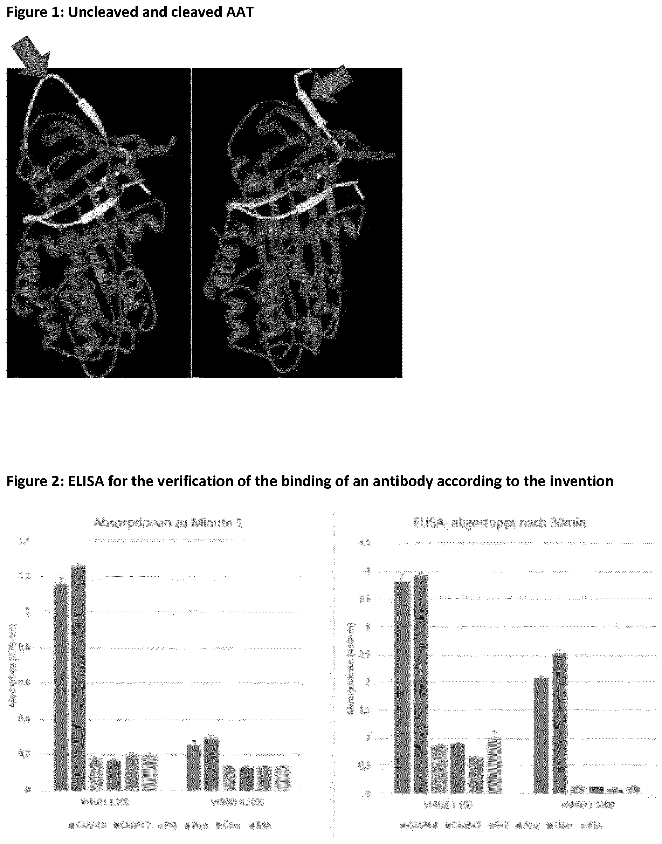

However, the quantitative detection of CAAP47/48 is quite difficult due to the structure and physical properties of the CAAP/AAT-complex (Fig. 1). The previously described LC-MS/MS method for example (Blaurock et al., Mediators of inflammation, 2016) is time consuming and therefore allows only for low sample throughput. Therefore, there is a need for new methods with improved diagnostic characteristics.

The inventors solved this problem by creating an antibody that recognizes CAAP47/48 fragments with high specificity and sensitivity and which can therefore be used in immunological assays for diagnostics.

The generation of such antibodies however, is not trivial and there are several problems which had not been solved before. One main problem for the production of such specific antibody is that after cleavage of AAT in the Reactive Center Loop (RCL), the fragment generated remains in a pocket of the AAT and is hardly released under physiological conditions (Fig. 1). Thus, the production of antibodies that specifically recognize CAAP but not AAT was up to date impossible. Commercially available antibodies bind both the total AAT and partially CAAP48 and are thus not suitable for the specific detection of CAAP47/48.

Aim of this work was the creation of an antibody, which specifically recognizes CAAP47/48, but not the rest of the AAT. The antibody according to the present invention was designed to have properties allowing for a broad range of applications in the biotechnical field as well as therapeutic use.

### Summary of the invention

Despite the difficulties in obtaining an antibody that is capable of specifically recognizing the antitrypsin (AAT) fragment CAAP48/47 but not AAT itself, the inventors were able to create such antibody by using the technology of phage display. The antibody according to the invention was designed to not only bind to CAAP48/47 with high specificity and affinity but also to be well expressed in a broad range of microorganisms and to possess a high stability and solubility. Moreover, due to its small size and high affinity it is an ideal agent for diagnostic and therapeutic use.

More precisely, the present invention relates to an anti-CAAP47/48 antibody or fragment or derivative thereof that contains at least a portion of said antibody that is sufficient to confer CAAP47/48 binding specificity, comprising a heavy chain variable region (VH) comprising the complementary determining regions comprising CDR-H1, CDR-H2, and CDR-H3, wherein the CDR-H1 region comprises an amino acid sequence selected from the group of SEQ ID NO: 4 - 6, wherein the CDR-H2 region comprises an amino acid sequence selected from the group of SEQ ID NO: 7 - 9, and wherein the CDR-H3 region comprises an amino acid sequence selected from the group of SEQ ID NO: 10 - 12.

The present invention further relates to a vector encoding said antibody and a kit comprising said antibody. The antibody can be used for determining and/or differentially diagnosing a CAAP47/48 correlated disease, such as a microbial infection. For example, the antibody according to the present invention can be used for determining whether an infection is of bacterial or non-bacterial origin.

Another aspect of the present invention is a method for monitoring the treatment of a patient with an antimicrobial drug, comprising determining the level of CAAP47/48 in a sample taking from said patient by using the antibody according to the invention and evaluating the effect of treatment with said antimicrobial drug based on the comparison of the levels of CAAP47/48 to reference values and the level of CAAP47/48 in samples taken at different time-points from the same patient.

### Definitions

In the context of the present application, the term "fragment" refers to a proteolytic cleavage product of a respective full-length protein.

The term "patient" as used herein refers to a living human or non-human organism that is receiving medical care or that should receive medical care due to a disease, or is suspected of having a disease. This includes persons with no defined illness who are being investigated for signs of pathology. Thus the methods and assays described herein are applicable to both, human and veterinary disease.

Herein, **"alpha-1-antitrypsin"** is also referred to as antitrypsin and **"AAT".** Alpha 1 Antitrypsin or α-1-antitrypsin (A1AT) is a protease inhibitor belonging to the serpin superfamily. It is generally known as serum trypsin inhibitor. Alpha-1-antitrypsin is also referred to as alpha-1-proteinase inhibitor (A1PI) because it inhibits a wide variety of proteases. It protects tissues from enzymes of inflammatory cells, especially neutrophil elastase, and has a reference range in blood of 1.5 - 3.5 g/l, but the concentration can rise manifold upon acute inflammation. AAT is cleaved at the reactive center loop (RCL) by target proteases (e.g. neutrophil elastase), as well as non-target proteases (e.g. matrix metalloproteases). Both reactions lead to a conformational change and a loss of inhibitory activity.

**C-terminal alpha-1 antitrypsin peptide** is herein referred to as **CAAP48.** CAAP48 is generated through the cleavage of AAT between Phe352-Leu353 and results in a carboxyl-terminal 42-residue peptide with a mass of 4789 Da, which is bound to the cleaved complex in a non-covalent manner. Variants of this fragment such as VIRIP or C-36 peptide have been identified in several human tissues and body fluids. For these C-terminal fragments physiological functions, such as NK-cell suppression and serine protease protection (CRISPP peptide), extracellular matrix protection by reversible serine protease inhibition (SPAAT), pro- or anti-inflammatory immune modulating functions (C-36 peptide) as well as inhibition of HIV entry (VIRIP) have been reported.

**CAAP47** is a SNP variant of CAAP48, resulting from a E > D substitution. In the AAT gene over 75 polymorphisms have been described and one frequent SNP (rs1303, MAF = 0.28) occurs in the sequence of CAAP48. Cleavage of AAT between Phe352-Leu353 in the presence of SNP rs1303 leads to the generation of a cleavage product with a lower molecular mass of 4775 Da, designated CAAP47.

In the context of the present invention, the terms "threshold", "threshold value", **"cut-off"** and "cut-off value" are used synonymously. The optimal cut-off value is defined to be the concentration at which patients are classified into the bacterial or non-bacterial group with the highest sensitivity and specificity.

As used herein, a "kit" is a packaged combination optionally including instructions for use of the combination and/or other reactions and components for such use.

The term **"antibody"** encompasses the various forms of antibody structures including, but not being limited to, whole antibodies and antibody fragments as long as it shows the properties according to the invention.

A **single-domain antibody** (sdAb) is an antibody fragment consisting of a single monomeric variable antibody domain. Like a whole antibody, it is able to bind selectively to a specific antigen. With a molecular weight of only 12-15 kDa, single-domain antibodies are much smaller than common antibodies (150-160 kDa) which are composed of two heavy protein chains and two light chains, and even smaller than Fab fragments (∼50 kDa, one light chain and half a heavy chain) and single-chain variable fragments (∼25 kDa, two variable domains, one from a light and one from a heavy chain). The first single-domain antibodies were engineered from heavy-chain antibodies found in camelids; these are called **VHH fragments.**

**"Percent (%) amino acid sequence identity"** with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software.

The term **"VL (or VH) region"** has the same meaning as VL (or VH) domain. It refers to the variable light or variable heavy chain region, respectively.

The term "specifically binding" or "specifically recognized" herein means that an antibody exhibits appreciable affinity for an antigen and, preferably, does not exhibit significant cross-reactivity.

The term "antigen-binding portion of an antibody" when used herein refers to the amino acid residues of an antibody which are responsible for antigen-binding. The antigen-binding portion of an antibody comprises preferably amino acid residues from the "complementary determining regions" or "CDRs". The CDR sequences are defined according to Kabat et al, Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a FR (framework region) or CDR of the variable region.

### Detailed description of the invention

The timely discrimination of a bacterial from a non-bacterial infection is essential for the initiation of an early goal directed therapy. In previous studies the inventors identified CAAP48 and the SNP variant CAAP47 (rs1303) as ideal biomarkers for that. CAAP47/48 is a C-terminal proteolytic cleavage product of Alpha-1 antitrypsin (AAT), which is increasingly formed at inflammation sites. Concentration measurements and analysis of AAT and its fragments revealed an increased proteolytic activity in patients with bacterial infection resulting in high peak intensities of AAT fragments compared to infections of non-bacterial origin. However, the suitable assays for the specific detection of CAAP48/47 fragments (and not the full-length AAT) are still lacking up to date.

The present invention therefore relates to an antibody for the specific detection of CAAP48/47 fragments which can be used in a variety of immunological assays. The useful properties of the antibody according to the invention also allow for a wide range of other applications in the biotechnical field, e.g. also for *in vivo* diagnosis using imaging as well as therapeutic use.

Despite the difficulties in obtaining an antibody that is capable of specifically recognizing the antitrypsin (AAT) fragment CAAP48/47 but not AAT itself, the inventors were able to create such antibody from a phage library of cameloid antibodies. The antibody according to the invention binds to CAAP48/47 with high specificity and affinity and can be well expressed in a broad range of microorganisms.

More specifically, the antibody according to the invention is an anti-CAAP47/48 antibody or fragment or derivative thereof that contains at least a portion of said antibody that is sufficient to confer CAAP47/48 binding specificity, comprising a heavy chain variable region (VH) comprising the complementary determining regions comprising CDR-H1, CDR-H2, and CDR-H3, wherein the CDR-H1 region comprises an amino acid sequence selected from the group of SEQ ID NO: 4 - 6, wherein the CDR-H2 region comprises an amino acid sequence selected from the group of SEQ ID NO: 7 - 9, and wherein the CDR-H3 region comprises an amino acid sequence selected from the group of SEQ ID NO: 10 - 12.

In a preferred embodiment the Anti-CAAP47/48 antibody or fragment or derivative thereof, comprises the heavy chain CDR1, CDR2, and CDR3 sequences selected from SEQ ID NO: 4, SEQ ID NO: 7, SEQ ID NO: 10, respectively; SEQ ID NO: 5, SEQ ID NO: 8, SEQ ID NO: 11, respectively; and SEQ ID NO: 6, SEQ ID NO: 9, SEQ ID NO: 12, respectively.

The present invention also relates to an anti-CAAP47/48 antibody or fragment or derivative thereof that contains at least a portion of said antibody that is sufficient to confer CAAP47/48 binding specificity, wherein the antibody comprises a heavy chain variable (VH) region that is at least 60% identical, preferably at least 70% identical, more preferably at least 80% identical, more preferably at least 90 % identical to a VH region selected from the group consisting of VH regions of SEQ ID NO: 1 to 3.

Preferred is an anti-CAAP47/48 antibody or fragment or derivative thereof that contains at least a portion of said antibody that is sufficient to confer CAAP47/48 binding specificity, wherein the antibody comprises a heavy chain variable (VH) region that is at least 91%, 92%, 93%, 94%, 95%, 96%, 97%, 98%, 99%, or 100% identical to a VH region selected from the group consisting of VH regions of SEQ ID NO: 1 to 3.

Further preferred is an anti-CAAP47/48 antibody or fragment or derivative thereof, wherein the antibody comprises a heavy chain variable (VH) region selected from the group consisting of VH regions of SEQ ID NO: 1 to 3.

Particularly preferred is an anti-CAAP47/48 antibody or fragment or derivative thereof that contains at least a portion of said antibody that is sufficient to confer CAAP47/48 binding specificity, selected from the group of antibodies consisting of VHH2, VHH3, VHH9.

The present invention also relates to an anti-CAAP47/48 antibody or fragment or derivative thereof according to the invention, which is cloned into a vector comprising the pelB-transport sequence of SEQ ID NO. 13, the linker sequence of SEQ ID NO. 14, and the alkaline phosphatase sequence with 6xHis-tag SEQ ID NO. 15.

As described above, obtaining an antibody that is capable of specifically recognizing the antitrypsin (AAT) fragment CAAP48/47 but not AAT itself has shown to be quite difficult and up to date, there were no such antibodies available.

Therefore, the present invention also relates to an anti-CAAP47/48 antibody or fragment or derivative thereof that contains at least a portion of said antibody that is sufficient to confer CAAP47/48 binding specificity, but does not bind to full-length AAT (Alpha-1-Antitrypsin) protein.

In one embodiment, such an anti-CAAP47/48 antibody or fragment or derivative thereof that contains at least a portion of said antibody that is sufficient to confer CAAP47/48 binding specificity, but does not bind to full-length AAT (Alpha-1-Antitrypsin) protein, comprises a heavy chain variable (VH) region that is at least 60% identical, preferably at least 70% identical, more preferably at least 80% identical, more preferably at least 90 % identical to a VH region selected from the group consisting of VH regions of SEQ ID NO: 1 to 3.

Preferably, said anti-CAAP47/48 antibody or fragment or derivative thereof comprises a heavy chain variable (VH) region that is at least 60% identical, preferably at least 91%, 92%, 93%, 94%, 95%, 96%, 97%, 98%, 99%, or 100% identical to a VH region selected from the group consisting of VH regions of SEQ ID NO: 1 to 3.

Further preferred is that the anti-CAAP47/48 antibody or fragment or derivative thereof comprises a heavy chain variable (VH) region selected from the group consisting of VH regions of SEQ ID NO: 1 to 3.

In one embodiment the anti-CAAP47/48 antibody according to the invention comprises the VH region of SEQ ID NO: 3. In another embodiment the anti-CAAP47/48 antibody according to the invention comprises the VH region of SEQ ID NO: 2, and in another embodiment the anti-CAAP47/48 antibody according to the invention comprises the VH region of SEQ ID NO: 3.

VHH (single domain heavy chain antibody) libraries generated from immunized camelids retain full functional diversity. This contrasts with the diminished diversity of conventional antibody libraries because of reshuffling of VL and VH domains during library construction. As a result, high-affinity antigen-binding VHHs can be isolated.

Thus, in some embodiments according to the invention, the inventors created an anti-CAAP47/48 antibody or fragment or derivative thereof that is a camelid-derived antibody.

The antibody according to the invention is in some instances a single-domain antibody.

In such embodiments, the antibody according to the invention does not give side effects such as conventional antibodies. Conventional ("whole") antibodies can give side effects because of their bivalent nature, resulting in target cross-linking, or the presence of the Fc region. In embodiments according to the invention in which the antibody is a monovalent VHH, such side effects are not expected to occur.

Moreover, the antibody according to the invention was designed so that it can be economically produced in a variety of microorganisms and possesses a high stability. In some embodiments, the antibody according to the invention remains functional at 90°C or after incubation at high temperatures.

In some embodiments, the antibody according to the invention is also highly suited for expression as multivalent, including bispecific, formats or as enzyme fusions. This permits a plug-and-play approach, where, depending on the target, biology potency can be increased by multivalent constructs or bispecific VHH recognizing two different targets can be made. This also enables the tailor-made design of serum half-life using site-directed PEGylation or by targeting to long-lived serum proteins using bispecific VHHs.

The anti-CAAP47/48 antibody or fragment or derivative thereof according to invention can also be used in the diagnosis and/or treatment of a CAAP47/48 correlated disease.

In certain embodiments, said disease is selected from the group consisting of bacterial infection and non-bacterial infections. Here, the non-bacterial infections can be selected from the group of viral and fungal infections.

In another embodiment, said CAAP47/48 correlated disease is selected from the group consisting of initial sepsis, severe sepsis, septic shock, and SIRs.

The anti-CAAP47/48 antibody or fragment or derivative thereof according to the invention can also be used in distinguishing between different types of infection, wherein it is distinguished between at least two of the following infections: viral, bacterial and fungal infection.

In one embodiment, the anti-CAAP47/48 antibody or fragment or derivative thereof can also be used in monitoring the treatment of a patient with an antimicrobial drug. In some cases, the antimicrobial drug is an antibiotic.

The present invention also relates to a vector, encoding the sequence of an anti-CAAP47/48 antibody according to the invention.

In certain embodiments, such vector additionally comprises the sequences of a pel-B transport sequence, a linker and alkaline phosphatase with a 6xHis-tag according to SEQ ID NO: 13, 14 and 15, respectively.

The invention is also directed to the use of the antibody according to the invention for diagnosing a CAAP47/48 correlated disease.

Hereby, the disease can be selected from the group consisting of bacterial infection and non-bacterial infections. In case of a non-bacterial infections, it can be selected from the group of viral and fungal infections.

The CAAP47/48 correlated disease for which the antibody according to the invention is used in diagnosis, can also be selected from the group consisting of initial sepsis, severe sepsis, septic shock, SIRs.

In some embodiments, the antibody according to the invention is used for distinguishing between different types of infection, wherein it is distinguished between at least two of the following infections: viral, bacterial and fungal infection.

Concentration measurements of AAT and its fragments, in combination with the mindful analysis carried out by the inventors, surprisingly revealed an increased proteolytic activity in patients with bacterial infection resulting in high peak intensities of AAT fragments compared to infections of non-bacterial origin. The inventors also found that the concentration of ATT fragments, is also substantially lower in patients suffering from a fungal infection than in patients suffering from a bacterial infection.

Thus, in a use of the antibody according to the invention, an infection is designated as of bacterial origin if the concentration of CAAP47/48 is above a certain cut-off value. If the concentration of CAAP47/48 is below a certain cut-off value, the infection is designated as of non-bacterial origin.

Said non-bacterial origin can be, but is not limited to viral origin or fungal origin.

The optimal cut-off value is defined to be the concentration at which patients are classified into the bacterial or non-bacterial group with the highest sensitivity and specificity.

In one embodiment, the antibody according to the invention is also used for monitoring the treatment of a patient with an antimicrobial drug. In such case, the antimicrobial drug can be an antibiotic.

Furthermore, the present invention relates to a method of differentially diagnosing in a patient a disease in which the levels of CAAP47/48 are altered compared to a healthy individual or a patient suffering from a different disease, comprising determining the level of CAAP47/48 in a sample taking from said patient by using the antibody according to the invention, and diagnosing said disease based on the comparison of the levels of CAAP47/48 to reference values.

In some embodiments, the disease is selected from the group comprising bacterial, viral and fungal infections.

The inventors found that the concentration of CAAP47/48, is clearly lower in patients suffering from a viral infection than in patients suffering from a bacterial infection. The inventors also found that the concentration of CAAP47/48 is also substantially lower in patients suffering from a fungal infection than in patients suffering from a bacterial infection.

Consequently, the present invention relates to a method wherein, if the concentration of CAAP47/48 is below a certain cut-off value, the infection is designated as of non-bacterial origin. Said non-bacterial origin can be, but is not limited to viral origin or fungal origin.

In one embodiment of the method according to the invention, the infection is designated as of bacterial origin, if the concentration of CAAP47/48 is at least 2.5 -fold higher in the patient than a reference concentration. Preferably, the concentration is at least 3 -fold higher, more preferred, at least 4 -fold higher and most preferred at least 5 -fold higher than the reference concentration of CAAP47/48 thereof.

In one embodiment of the method according to the invention, the infection is designated as of non-bacterial origin if the concentration of CAAP47/48 is not more than 2-fold higher than a reference concentration. Preferably, said concentration is not more than 1.5 -fold higher than a reference concentration.

In other embodiments, the disease is selected from the group comprising sepsis, severe sepsis, septic shock and SIRS. In these cases, also specific cut-off values are used in order to differentially diagnose the type of inflammatory reaction.

According to the invention, a correlation of the level of CAAP47/48 to initial sepsis, severe sepsis or septic shock is given if the level of CAAP47/48 is above a certain cut-off value. Preferably, the cut-off value of CAAP47/48 is about 4,18 pmol/µl, and may deviate depending on the patient analyzed by about 5%, 10% or even 20%.

Due to the easy use, low running costs and capacity of high sample throughput, the method according to the invention can be used not only for determining if an infection is of bacterial or non-bacterial origin, but also for the monitoring of treatment of a patient. Such treatment may comprise the treatment of an infection such as a bacterial, viral or fungal infection. The monitoring of treatment can relate to the monitoring of treatment of a patient with an antimicrobial drug, such as antibiotics.

Thus, the method according to the invention can also be used to determine the appropriate treatment and/or therapy. For example, it can be used to determine the right medication before and during treatment.

Therefore, the present invention also relates to a method of monitoring the treatment of a patient with an antimicrobial drug, comprising determining the level of CAAP47/48 in a sample taking from said patient by using the antibody according to the invention, evaluating the effect of treatment with said antimicrobial drug based on the comparison of the levels of CAAP47/48 to reference values and the level of CAAP47/48 at different time-points in the same patient.

Thus, the method according to the invention may prevent harmful side-effects, toxicity and allergic reactions due to inappropriate drug treatment and/or therapy. Moreover, serious complications can be prevented in patients and the healing process can be accelerated. This may also significantly reduce the costs of therapy. In case of treating a patient presumably suffering from an infection, the inappropriate prescription of antibiotics may be prevented, thus preventing the spread of antibiotic resistance.

The invention also relates to a method of medical decision making for individual patient therapy in a subject suffering from an infection, comprising the steps of, determining the level of CAAP47/48 by using the antibody according to the invention, in a sample that has been taken from said patient, and associating the levels of CAAP47/48 with a certain choice of therapy. As example, it can be used to determine whether a patient should be treated with antibiotics.

The invention also relates to a method of medical decision making for individual patient therapy in a subject related to the severity of the disease. The method according to the invention comprises the monitoring therapy response in a subject to a certain drug. Such drug can be an antimicrobial-drug, e.g. antibiotics.

In one embodiment, the method according to the invention relates to a method for monitoring the success of treating a patient that suffering from an infection with a drug, comprising the steps of taking at least two samples from said patient at various time points selected from the group of,
i. before initiation of therapy and/or
ii. after initiation of therapy, and/or
iii. at one or more further time point
determining the concentration of CAAP47/48 in said sample taken from said patient by using an antibody according to the invention, and associating the levels of CAAP47/48 in said samples taken from said patient with a positive or a negative response to said certain drug.

Said positive response is given if the level of CAAP47/48 decreases during drug treatment.

In a method according to the invention, the sample can be selected from a group comprising a plasma sample, a serum sample, a whole blood sample, a blood sample or fractions thereof, a lymphatic fluid sample, a urine sample and an extract of any of the aforementioned samples.

The invention also relates to a kit for determining and /or differentially diagnosing a CAAP47/48 correlated disease, or for monitoring the treatment of a patient with an antimicrobial drug, comprising:
(a) an anti-CAAP47/48 antibody or fragment or derivative thereof according to the invention;
(b) means for comparing the determined concentration of said CAAP47/48 with reference concentrations; and
(c) instructions for carrying out the method and correlating the determined concentrations with the type of disease.

In some embodiments, the antimicrobial drug is an antibiotic.

In another embodiment, the means for comparing the determined concentrations comprise standard data showing the correlation between the concentration of CAAP47/48 contained in samples and the origin of infection.

The present invention also relates to a kit as described above, wherein said infection of non-bacterial origin is an infection selected from the group comprising viral and fungal infections.

The present invention also relates to a kit according to the invention, wherein said sample is selected from a group comprising a plasma sample, a serum sample, a whole blood sample, a blood sample or fractions thereof, a lymphatic fluid sample, a urine sample and an extract of any of the aforementioned samples.

The kit according to the invention can be used in the differential diagnoses of a disease in a patient in which the levels of CAAP47/48 are altered compared to a healthy individual or a patient suffering from a different disease.

The kit can be used for medical decision making in individual patient therapy in a subject suffering from an infection. For example, it can be used to determine whether a patient should be treated with antibiotics.

The kit according to the invention can also be used in the medical decision making for individual patient therapy in a subject related to the severity of the disease.

In another embodiment, the kit is used in the monitoring therapy response in a subject to a certain drug. Such drug can be an antimicrobial-drug, e.g. antibiotics.

### Examples

The following examples are used in conjunction with the figures and tables to illustrate the invention.

### Example 1: Generation of antibody

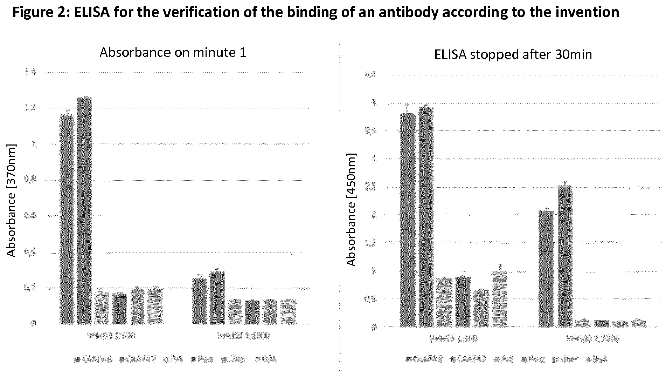

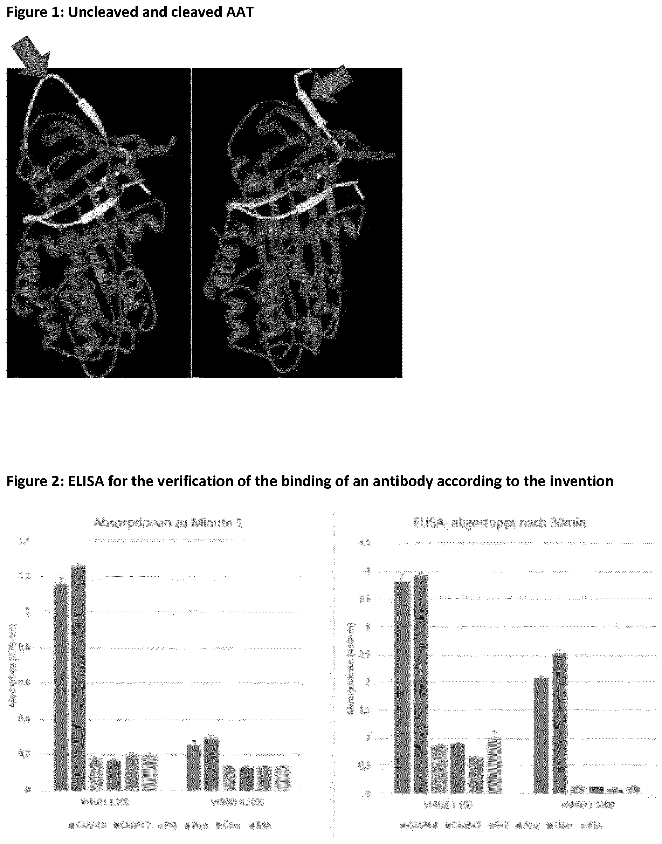

A phage library of camelid antibodies was screened for antibody chains with particularly high CAAP47/48 binding capacity. Randomly generated variable antibody chains (VHH sequences), bound to bacteriophages were screened by phage display. The genetic information of the respective antibody chains is contained in the phage capsule. As bait, synthetically generated CAAP48 bound to a ELISA plate was used (Fig. 2).

If an antibody chain with respective bacteriophage was bound to CAAP48, it was copied in *E. coli* and the DNA with the information for respective antibody was isolated. The DNA was cloned in suitable vectors, expressed in *E. coli* and subsequently purified. In some instances, the vector contained additional information for a flag-tag, a His-tag and an alkaline phosphatase (AP) for later characterization of the antibody.

Phage clones that showed high CAAP48 binding affinity and specificity were sequenced, synthesized *de novo,* cloned in vectors with flag-tag, his-tag and AP and finally expressed in *E. coli.*

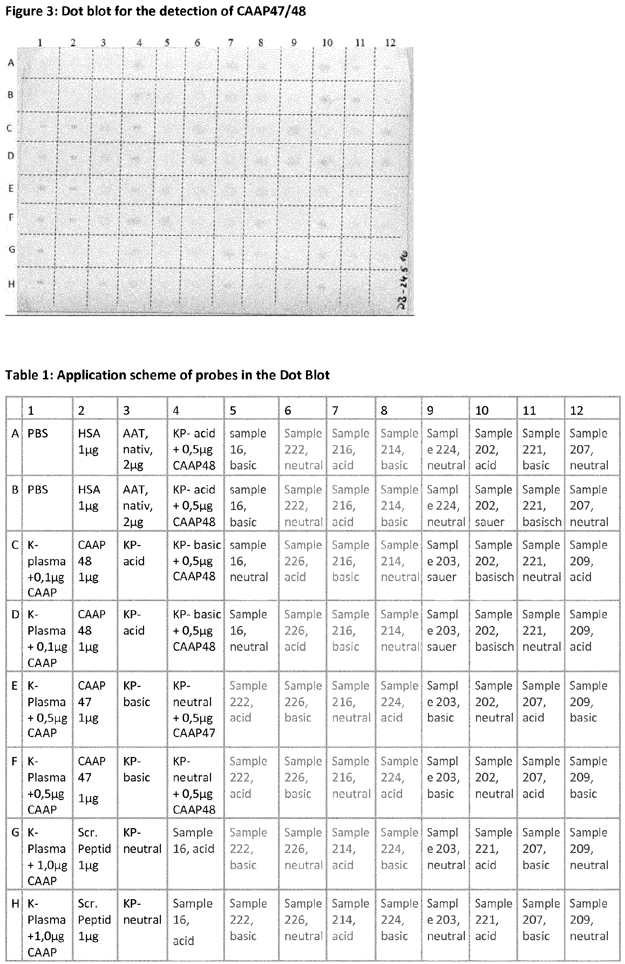

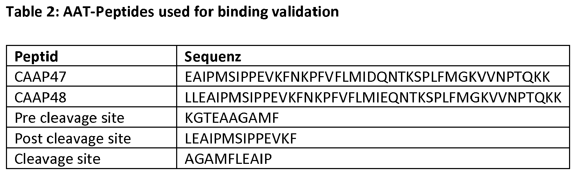

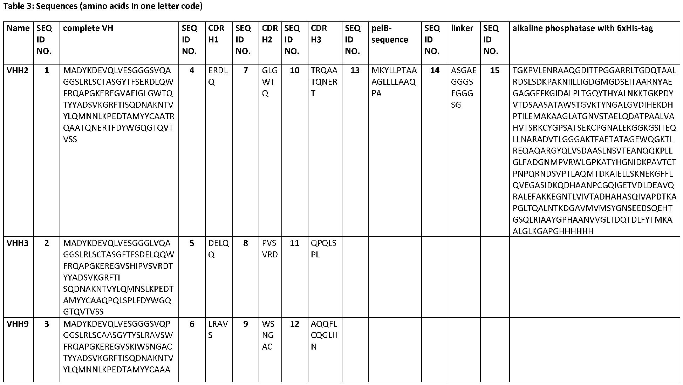

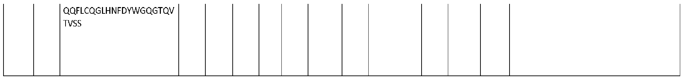

Specific binding was subsequently validated in Dot Blot experiments (Fig. 3 and Table 1).

### Figure legend

**Figure 1****: Uncleaved and cleaved AAT**
   CAAP47/48 (in white, arrows pointing) is embedded into the surrounding AAT and is released to certain extend into the inflamed microclimate. The left picture shows uncleaved AAT, the right picture shows cleaved AAT. Aim of this invention was the creation of a specific antibody that detects CAAP47/48 but not the surrounding AAT.
**Figure 2****: ELISA to verify specific binding of an antibody according to the invention**
   Results of ELISA experiments showing that the antibody according to the invention specifically binds to CAAP47 and CAAP48, but not the cleavage site or unspecifically to BSA.
   Column 1: CAAP 48
   Column 2: CAAP 47
   Column 3: Pre-cleavage site
   Column 4: Post-cleavage site
   Column 5: Cleavage Site
   Column6: BSA
**Figure 3****: Dot blot for the detection of CAAP47/48**
   Dot Blot showing the specific detection of CAAP47/48 fragments in the plasma of patients using an antibody according to the invention. The antibody according to the invention binds specifically to CAAP 47 and 48 but not unspecifically to BSA. The scheme of application of probes in the Dot Blot is shown in Table 1.
**Table 1: Application scheme of probes in the Dot Blot**
   Probes as applied in dot plot experiments are indicated in the boxes.
**Table 2: AAT-Peptides used for binding validation**
   Shown are the amino acid sequences in one letter code.

**Table 3: Sequences (amino acids in one letter code)**

| | |
|---|---|
| VH complete: | SEQ ID NO: 1-3 |
| CDR-H1: | SEQ ID NO: 4-6 |
| CDR-H2: | SEQ ID NO: 7-9 |
| CDR-H3: | SEQ ID NO: 10-12 |
| PeIB: | SEQ ID NO: 13 |
| Linker: | SEQ ID NO: 14 |
| Phosphatase: | SEQ ID NO: 15 |

## Claims

1. An anti-CAAP47/48 antibody or fragment or derivative thereof that contains at least a portion of said antibody that is sufficient to confer CAAP47/48 binding specificity, comprising:
a heavy chain variable region (VH) comprising the complementary determining regions (CDRs) comprising CDR-H1, CDR-H2, and CDR-H3
(i) wherein the CDR-H1 region comprises an amino acid sequence selected from the group of SEQ ID NO: 4 - 6,
(ii) wherein the CDR-H2 region comprises an amino acid sequence selected from the group of SEQ ID NO: 7 - 9, and
(iii) wherein the CDR-H3 region comprises an amino acid sequence selected from the group of SEQ ID NO: 10-12,
wherein the CDRs may comprise any one or more amino acid mutations that does not diminish their activity according to the invention.

2. Anti-CAAP47/48 antibody or fragment or derivative thereof according to claim 1, comprising the heavy chain CDR1, CDR2, and CDR3 sequences selected from:
(i) SEQ ID NO: 4, SEQ ID NO: 7, SEQ ID NO: 10, respectively;
(ii) SEQ ID NO: 5, SEQ ID NO: 8, SEQ ID NO: 11, respectively; and
(iii) SEQ ID NO: 6, SEQ ID NO: 9, SEQ ID NO: 12, respectively,
wherein the CDRs may comprise any one or more amino acid mutations that does not diminish their activity according to the invention.

3. Anti-CAAP47/48 antibody or fragment or derivative thereof that contains at least a portion of said antibody that is sufficient to confer CAAP47/48 binding specificity, wherein the antibody comprises a heavy chain variable (VH) region that is at least 90% identical to a VH region selected from the group consisting of VH regions of SEQ ID NO: 1 to 3.

4. Anti-CAAP47/48 antibody or fragment or derivative thereof that contains at least a portion of said antibody that is sufficient to confer CAAP47/48 binding specificity, but does not bind to full-length AAT (Alpha-1-Antitrypsin) protein.

5. Anti-CAAP47/48 antibody or fragment or derivative thereof according to claim 4, comprising a heavy chain variable (VH) region that is at least 90% identical to a VH region selected from the group consisting of VH regions of SEQ ID NO: 1 to 3.

6. Anti-CAAP47/48 antibody or fragment or derivative thereof according to any of the previous claims, wherein the antibody is a camelid-derived antibody.

7. Anti-CAAP47/48 antibody or fragment or derivative thereof according to any of the previous claims, for use in the diagnosis of a CAAP47/48 correlated disease.

8. Anti-CAAP47/48 antibody according to claim 7, wherein the disease is selected from the group consisting of bacterial infection and non-bacterial infections,

9. Anti-CAAP47/48 antibody according to claim 8, wherein the non-bacterial infections are selected from the group of viral and fungal infections.

10. Anti-CAAP47/48 antibody according to claim 7 or 8, wherein the disease is selected from the group consisting of initial sepsis, severe sepsis, septic shock, and SIRs.

11. Anti-CAAP47/48 antibody or fragment or derivative thereof according to any of the claims 1 to 10, for the use in distinguishing between different types of infection, wherein it is distinguished between at least two of the following infections: viral, bacterial and fungal infection.

12. Anti-CAAP47/48 antibody or fragment or derivative thereof according to claims 1 to 6 for the use in monitoring the treatment of a patient with an antimicrobial drug.

13. Vector, comprising the sequence of an anti-CAAP47/48 antibody according to any of the previous claims.

14. Use of the antibody according to any of the claims 1-10 for diagnosing a CAAP47/48 correlated disease and/or for distinguishing between different types of infection

15. A kit for determining and/or differentially diagnosing a CAAP47/48 correlated disease, or for monitoring the treatment of a patient with an antimicrobial drug, comprising:
(a) an anti-CAAP47/48 antibody or fragment or derivative thereof according to any of the claims 1 to 10;
(b) means for comparing the determined concentration of said CAAP47/48 with reference concentrations; and
(c) instructions for carrying out the method and correlating the determined concentrations with the type of disease.


SEQUENCE LISTING

<110>  Universitätsklinikum Jena

<120>  Antibody for the specific detection of CAAP47/48 fragments

<130>  B134-0004EP1

<160>  15

<170>  PatentIn version 3.5

<210>  1
<211>  129
<212>  PRT
<213>  Camelus bactrianus

<400>  1

Met Ala Asp Tyr Lys Asp Glu Val Gln Leu Val Glu Ser Gly Gly Gly
1               5                   10                  15

Ser Val Gln Ala Gly Gly Ser Leu Arg Leu Ser Cys Thr Ala Ser Gly
            20                  25                  30

Tyr Thr Phe Ser Glu Arg Asp Leu Gln Trp Phe Arg Gln Ala Pro Gly
        35                  40                  45

Lys Glu Arg Glu Gly Val Ala Glu Ile Gly Leu Gly Trp Thr Gln Thr
    50                  55                  60

Tyr Tyr Ala Asp Ser Val Lys Gly Arg Phe Thr Ile Ser Gln Asp Asn
65                  70                  75                  80

Ala Lys Asn Thr Val Tyr Leu Gln Met Asn Asn Leu Lys Pro Glu Asp
                85                  90                  95

Thr Ala Met Tyr Tyr Cys Ala Ala Thr Arg Gln Ala Ala Thr Gln Asn
            100                 105                 110

Glu Arg Thr Phe Asp Tyr Trp Gly Gln Gly Thr Gln Val Thr Val Ser
        115                 120                 125

Ser

<210>  2
<211>  125
<212>  PRT
<213>  Camelus bactrianus

<400>  2

Met Ala Asp Tyr Lys Asp Glu Val Gln Leu Val Glu Ser Gly Gly Gly

1                   5                   10                  15

Leu Val Gln Ala Gly Gly Ser Leu Arg Leu Ser Cys Thr Ala Ser Gly
            20                  25                  30

Phe Thr Phe Ser Asp Glu Leu Gln Gln Trp Phe Arg Gln Ala Pro Gly
        35                  40                  45

Lys Glu Arg Glu Gly Val Ser His Ile Pro Val Ser Val Arg Asp Thr
    50                  55                  60

Tyr Tyr Ala Asp Ser Val Lys Gly Arg Phe Thr Ile Ser Gln Asp Asn
65                  70                  75                  80

Ala Lys Asn Thr Val Tyr Leu Gln Met Asn Ser Leu Lys Pro Glu Asp
                85                  90                  95

Thr Ala Met Tyr Tyr Cys Ala Ala Gln Pro Gln Leu Ser Pro Leu Phe
            100                 105                 110

Asp Tyr Trp Gly Gln Gly Thr Gln Val Thr Val Ser Ser
        115                 120                 125

<210>  3
<211>  129
<212>  PRT
<213>  Camelus bactrianus

<400>  3

Met Ala Asp Tyr Lys Asp Glu Val Gln Leu Val Glu Ser Gly Gly Gly
1               5                   10                  15

Ser Val Gln Pro Gly Gly Ser Leu Arg Leu Ser Cys Ala Ala Ser Gly
            20                  25                  30

Tyr Thr Tyr Ser Leu Arg Ala Val Ser Trp Phe Arg Gln Ala Pro Gly
        35                  40                  45

Lys Glu Arg Glu Gly Val Ser Lys Ile Trp Ser Asn Gly Ala Cys Thr
    50                  55                  60

Tyr Tyr Ala Asp Ser Val Lys Gly Arg Phe Thr Ile Ser Gln Asp Asn
65                  70                  75                  80

Ala Lys Asn Thr Val Tyr Leu Gln Met Asn Asn Leu Lys Pro Glu Asp
                85                  90                  95

Thr Ala Met Tyr Tyr Cys Ala Ala Ala Gln Gln Phe Leu Cys Gln Gly

                100                 105                 110

Leu His Asn Phe Asp Tyr Trp Gly Gln Gly Thr Gln Val Thr Val Ser
        115                 120                 125

Ser

<210>  4
<211>  5
<212>  PRT
<213>  Camelus bactrianus

<400>  4

Glu Arg Asp Leu Gln
1               5

<210>  5
<211>  5
<212>  PRT
<213>  Camelus bactrianus

<400>  5

Asp Glu Leu Gln Gln
1               5

<210>  6
<211>  5
<212>  PRT
<213>  Camelus bactrianus

<400>  6

Leu Arg Ala Val Ser
1               5

<210>  7
<211>  6
<212>  PRT
<213>  Camelus bactrianus

<400>  7

Gly Leu Gly Trp Thr Gln
1               5

<210>  8
<211>  6
<212>  PRT
<213>  Camelus bactrianus

<400>  8

Pro Val Ser Val Arg Asp

1                5

<210>  9
<211>  6
<212>  PRT
<213>  Camelus bactrianus

<400>  9

Trp Ser Asn Gly Ala Cys
1               5

<210>  10
<211>  11
<212>  PRT
<213>  Camelus bactrianus

<400>  10

Thr Arg Gln Ala Ala Thr Gln Asn Glu Arg Thr
1               5                   10

<210>  11
<211>  7
<212>  PRT
<213>  Camelus bactrianus

<400>  11

Gln Pro Gln Leu Ser Pro Leu
1               5

<210>  12
<211>  11
<212>  PRT
<213>  Camelus bactrianus

<400>  12

Ala Gln Gln Phe Leu Cys Gln Gly Leu His Asn
1               5                   10

<210>  13
<211>  11
<212>  PRT
<213>  Camelus bactrianus

<400>  13

Ala Gln Gln Phe Leu Cys Gln Gly Leu His Asn
1               5                   10

<210>  14
<211>  15
<212>  PRT
<213>  Camelus bactrianus

<400>  14

Ala Ser Gly Ala Glu Gly Gly Gly Ser Glu Gly Gly Gly Ser Gly
1               5                   10                  15


<210>  15
<211>  458
<212>  PRT
<213>  Camelus bactrianus

<400>  15

Thr Gly Lys Pro Val Leu Glu Asn Arg Ala Ala Gln Gly Asp Ile Thr
1               5                   10                  15


Thr Pro Gly Gly Ala Arg Arg Leu Thr Gly Asp Gln Thr Ala Ala Leu
            20                  25                  30


Arg Asp Ser Leu Ser Asp Lys Pro Ala Lys Asn Ile Ile Leu Leu Ile
        35                  40                  45


Gly Asp Gly Met Gly Asp Ser Glu Ile Thr Ala Ala Arg Asn Tyr Ala
    50                  55                  60


Glu Gly Ala Gly Gly Phe Phe Lys Gly Ile Asp Ala Leu Pro Leu Thr
65                  70                  75                  80


Gly Gln Tyr Thr His Tyr Ala Leu Asn Lys Lys Thr Gly Lys Pro Asp
                85                  90                  95


Tyr Val Thr Asp Ser Ala Ala Ser Ala Thr Ala Trp Ser Thr Gly Val
            100                 105                 110


Lys Thr Tyr Asn Gly Ala Leu Gly Val Asp Ile His Glu Lys Asp His
        115                 120                 125


Pro Thr Ile Leu Glu Met Ala Lys Ala Ala Gly Leu Ala Thr Gly Asn
    130                 135                 140


Val Ser Thr Ala Glu Leu Gln Asp Ala Thr Pro Ala Ala Leu Val Ala
145                 150                 155                 160


His Val Thr Ser Arg Lys Cys Tyr Gly Pro Ser Ala Thr Ser Glu Lys
                165                 170                 175


Cys Pro Gly Asn Ala Leu Glu Lys Gly Gly Lys Gly Ser Ile Thr Glu
            180                 185                 190


Gln Leu Leu Asn Ala Arg Ala Asp Val Thr Leu Gly Gly Gly Ala Lys
        195                 200                 205

Thr Phe Ala Glu Thr Ala Thr Ala Gly Glu Trp Gln Gly Lys Thr Leu
    210                 215                 220

Arg Glu Gln Ala Gln Ala Arg Gly Tyr Gln Leu Val Ser Asp Ala Ala
225                 230                 235                 240

Ser Leu Asn Ser Val Thr Glu Ala Asn Gln Gln Lys Pro Leu Leu Gly
                245                 250                 255

Leu Phe Ala Asp Gly Asn Met Pro Val Arg Trp Leu Gly Pro Lys Ala
            260                 265                 270

Thr Tyr His Gly Asn Ile Asp Lys Pro Ala Val Thr Cys Thr Pro Asn
        275                 280                 285

Pro Gln Arg Asn Asp Ser Val Pro Thr Leu Ala Gln Met Thr Asp Lys
    290                 295                 300

Ala Ile Glu Leu Leu Ser Lys Asn Glu Lys Gly Phe Phe Leu Gln Val
305                 310                 315                 320

Glu Gly Ala Ser Ile Asp Lys Gln Asp His Ala Ala Asn Pro Cys Gly
                325                 330                 335

Gln Ile Gly Glu Thr Val Asp Leu Asp Glu Ala Val Gln Arg Ala Leu
            340                 345                 350

Glu Phe Ala Lys Lys Glu Gly Asn Thr Leu Val Ile Val Thr Ala Asp
        355                 360                 365

His Ala His Ala Ser Gln Ile Val Ala Pro Asp Thr Lys Ala Pro Gly
    370                 375                 380

Leu Thr Gln Ala Leu Asn Thr Lys Asp Gly Ala Val Met Val Met Ser
385                 390                 395                 400

Tyr Gly Asn Ser Glu Glu Asp Ser Gln Glu His Thr Gly Ser Gln Leu
                405                 410                 415

Arg Ile Ala Ala Tyr Gly Pro His Ala Ala Asn Val Val Gly Leu Thr
            420                 425                 430

Asp Gln Thr Asp Leu Phe Tyr Thr Met Lys Ala Ala Leu Gly Leu Lys
        435                 440                 445

Gly Ala Pro Gly His His His His His His

450                                                455